# EUROPEAN PATENT SPECIFICATION

(11) **EP 3 107 607 B1**
(45) Date of publication and mention of the grant of the patent: **01.04.2020**
(21) Application number: 15707068.1
(22) Date of filing: 19.02.2015
(51) Int. Cl.: A61M 15/06, A61M 11/04, A24F 47/00

(54) **LED MODULE FOR AEROSOL GENERATING DEVICES, AEROSOL GENERATING DEVICE HAVING A LED MODULE AND METHOD FOR ILLUMINATING VAPOUR**
LED-MODUL FÜR AEROSOLERZEUGENDE VORRICHTUNGEN, AEROSOLERZEUGENDE VORRICHTUNG MIT LED-MODUL UND VERFAHREN ZUR BELEUCHTUNG VON DAMPF
MODULE À DEL POUR DISPOSITIFS DE GÉNÉRATION D'AÉROSOL, DISPOSITIF DE GÉNÉRATION D'AÉROSOL AYANT UN MODULE À DEL ET PROCÉDÉ POUR ÉCLAIRER LA VAPEUR

(30) Priority: 20.02.2014 EP 14155969
(43) Date of publication of application: 28.12.2016
(73) Proprietor: JT International S.A., 1202 Geneva (CH)
(72) Inventor: PLATTNER, Michael, 54290 Trier (DE)
(74) Representative: Isarpatent
(86) International application number: PCT/EP2015/053527
(87) International publication number: WO 2015/124688

(56) References cited:
- WO-A1-99/37347
- WO-A1-2009/013843
- WO-A1-2009/115197
- WO-A1-2013/098334
- CN-A- 103 416 852
- US-A1- 2008 223 953
- US-A1- 2011 036 346
- US-A1- 2011 277 764
- US-A1- 2013 019 887

## Description

### Field of the invention

The present invention pertains to an LED module for aerosol generating devices, an aerosol generating device having an LED module and a method for illuminating vapour using an aerosol generating device equipped with an LED module.

### Background of the invention

Conventional electrically operated cigarettes, so-called "e-cigarettes", usually include a heater powered by an electrical power source and a liquid reservoir containing flavoured liquid that can be volatilized using the heater and transferred to a user of the e-cigarette in an airflow through a mouthpiece of the e-cigarette. Such an electrically operated cigarette is for example known from the document US 2013/0160764 A1.

Electrically operated cigarettes and other aerosol or steam generating devices produce vapour, i.e. particles of moisture or other substances suspended in air which are visible as clouds, steam or smoke. The vapour droplets have light scattering surfaces which act as source of outgoing spherical waves when illuminated with white light, thus having a generally white visual appearance. Document US 2008/0023003 A1 discloses a portable vaporizer having LED lamps in the housing to provide visual feedback of the vaporization status of the smokable substances. Document US 7,542,664 B2 discloses vapour dispensing devices having integrated light sources. Document WO 2009/115197 A1 discloses a vapour generating device having LED lights illuminating the generated vapour in different colours depending on the intended effect of the vapour.

US 2008/223963 A1 describes a mist generator including an ultrasonic transducer configured to atomize a liquid by use of ultrasonic waves. WO 2009/013843 A1 describes a portable ultrasonic mist generating device. US 2011/036346 A1 describes personal inhalation devices. WO 2009/115197 A1 describes an epilation device having a vaporizer unit adapted to vaporize an application material. The documents CN 103 416 852 A, US 2013/019887 A1 and US 2011/277764 A1 disclose some prior art e-cigarettes.

### Summary of the invention

The invention provides a component for an aerosol generating e-cigarette or a heat-not-burn tobacco aerosol generating device as defined in claim 1, and a method for illuminating vapour as defined in claim 7. Preferred embodiments of the invention are set out in the dependent claims.

It is one object of the present invention to provide controllable illumination of vapour particles, especially exhaled vapour when using an electrically operated aerosol generating device and improve interactivity with the user and its environment.

### Embodiments of the invention

One main idea of the preferred embodiment of the present invention is to provide one or more LEDs of controllable or predeterminable emission wavelength that can be added to or integrated in an aerosol generating device such as an electrically operated cigarette or vaporizer and that emits light which may be used to illuminate vapour particles, for example exhaled vapour from an aerosol generating device or a vapour stream in an aerosol generating device.

The LED or LEDs may emit light of a wavelength or wavelengths that may get diffracted and/or diffused by the vapour droplets, thereby emitting light of certain spectral colours. The colour depends on the size and/or shape of the droplets, on the chemical composition of the vapour as well as the wavelength of light emitted by the LED or LEDs.

According to an embodiment, the at least one LED is configured to emit light having a wavelength of 480 nm.

According to a further embodiment, the particle size of the vapour particles generated by the aerosol generating device is measured and the wavelength of the emitted light is adjusted depending on the measured particle size.

The invention will be explained in greater detail with reference to examples depicted in the drawings as appended.

### Brief description of the drawings

The accompanying drawings are included to provide a further understanding of the present invention and are incorporated in and constitute a part of this specification.

The drawings illustrate examples which together with the description serve to explain the principles of the invention.

The elements of the drawings are not necessarily to scale relative to each other. Like reference numerals designate corresponding similar parts.
Fig. 1 schematically illustrates a concept of an aerosol generating device.
Fig. 2A schematically illustrates an LED module for an aerosol generating device.
Fig. 2B schematically illustrates a component of an aerosol generating device having an integrated LED module.
Fig. 2C schematically illustrates a component of an aerosol generating device having two LED modules.
Fig. 3 schematically illustrates a method for illuminating vapour generated by an aerosol generating device.

### Detailed description of the embodiments

Although specific embodiments have been illustrated and described herein, it will be appreciated by those of ordinary skill in the art that a variety of alternate and/or equivalent implementations may be substituted for the specific embodiments shown and described without departing from the scope of the present invention as defined by the appended claims.

Generally, this application is intended to cover any adaptations or variations of the specific embodiments discussed herein. In the present disclosure, reference is made to aerosol generators. Such aerosol generators or aerosol generating device are generally intended to comprise any apparatus capable of converting electric energy and/or combustion energy into heat and subsequently heating and thereby volatilizing particles in a vaporisable material, for example, a liquid composition contained within a part of the aerosol generating device. Aerosol generating devices within the meaning of the present invention may transport the volatilized particles in an airflow through the aerosol generating device to a user of the device, the user of the device being able to activate or deactivate the generation of aerosol and to control the duration, velocity and volume of the airflow by means of puffing or inhaling action.

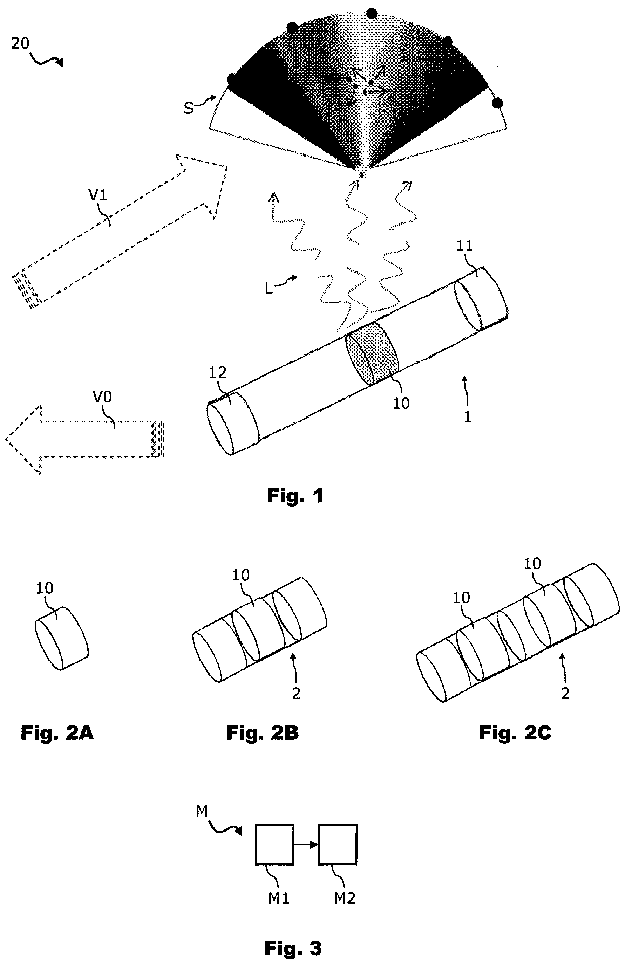

Fig. 1 schematically illustrates a user environment 20 of a user of an aerosol generating device 1. The aerosol generating device 1 generally comprises a power source, such as an electrical power source, for example a battery or an accumulator. The power source is operatively coupled to a heating element 11, the heating element 11 being supplied with energy for its operation by the power source. The power source and the heating element 11 may be part of a basis of the aerosol generating device 1. The aerosol generating device 1 may further comprise electronic circuitry controlling the operation of the aerosol generating device 1 and/or various buttons on its outer surface, all of which are not shown in Fig. 1 for purposes of improved clarity of the drawings.

The aerosol generating device 1 further comprises a storage component for housing an aerosol generating substance such as a liquid, a gas, or solid material capable of generating an aerosol or vapour upon heating. To that aim, the storage component (not explicitly shown in the drawings) is coupled to the heating element 11 in operative connection, so that the heating element 11 may supply heating energy to an aerosol generating substance supplied from the storage component, thereby causing certain compounds contained in the aerosol generating substance to volatilize into an vapour streaming through aerosol generating device 1, inside or outside the storage component. The aerosol generating device further comprises a mouthpiece 12 connected to the device 1 for a user to draw and to inhale the generated aerosol.

The overall arrangement of the power source, the heating element 11, the storage component, and the mouthpiece 12 may deviate from the conceptual illustration in Fig. 1 and may be adapted to the design and purpose of the aerosol generating device 1.

When an aerosol generating composition with compounds to be volatilized is heated up by heating energy from the heating element 11, certain compounds thereof evaporates or volatilize, thereby producing an aerosol stream. The aerosol or vapour stream V0 then exits the aerosol generating device 1 through the mouthpiece 12. The aerosol or vapour stream V0 may then be inhaled by the user in a smoking action and exhaled again as vapour stream V1. Furthermore, the vapour stream V1 may also consist of a part of the vapour stream V0 not having been inhaled by the user.

The vapour streams V0 and V1 comprise particles that - upon interaction with light - may transmit and or reflect light in such a way that the transmitted waves combine to form a diffraction pattern of a certain colour. Depending on the chemical and physical composition of the particles contained within the vapour stream V1, the particles emit light due to refraction and an effective appearance of light of an associated colour.

The aerosol generating device 1 comprises a light emitting device 10, such as a light emitting diode (LED) module 10 as integrated component of the aerosol generating device 1, for example incorporated into the housing of the aerosol generating device 1. The light emitting device 10 may have a variety of different light generating device installed therein, however, in the following reference will be specifically made to LED modules, without loss of generality. The LED module 10 comprises at least one LED which is able to emit light rays L of a wavelength within a specific wavelength range of the visible spectrum S. According to the present invention, the LED module is chosen such as the emitted light rays L has a wavelength comprised between 465 nm and 485 nm.This specific wavelength range has been determined by the inventor as the one where light is most effectively scattered by vapour particles from aerosol generating devices and in particular vapour produced from electronic cigarettes or heat-not-burn tobacco aerosol generating devices.

According to an embodiment of the present invention, the wavelength range is preferably chosen and can be tuned dependent on the particle size of the vapour particles contained within the vapour stream V1 produced by the aerosol generating device 1. The wavelength range may be set between 465 nm and 485 nm, and preferably about 480nm. Specifically, the wavelength of the light emitted by the at least one LED of the LED module 10 may be 465 nm, 470 nm, 475 nm, 480 nm or 485 nm.

Fig. 2A shows a schematic illustration of an LED module 10 adapted to be connected to an aerosol generating device, such as an aerosol generating device 1 as shown and explained in Fig. 1. The LED module 10 may comprise at least one LED. It may for example be possible to provide more than one LED in the LED module, with the different LEDs being capable of emitting light rays of different wavelengths in the range between 465 nm and 485 nm.

The LED module 10 may comprise one or more LEDs which are arranged in a substantially cylindrical housing. The LEDs are configured to emit light substantially perpendicular to an external and/or internal surface of the cylindrical housing, for example in a fan-shaped light curtain. Vapour travelling through this light curtain may be illuminated in the sectional plane of the fan with the contour of the vapour stream V1 in and/or outside the aerosol generating device 1 comprising the LED module 10.

The LED module 10 may further comprise means for altering optical diffusion and/or transmission of the light rays emitted by the LED.

In further embodiments of the invention, it may be possible to provide the housing with a partially translucent surface, such that light emitted by the LED or LEDs may only be partially transmitted to the surrounding. For example, it may be possible to provide a structured pattern on the partially translucent housing surface in order to create a patterned light output of the LED module 10. The structured pattern may for example be a masking pattern having different shapes such as initials of the name of the user, a picture of the e-cigarette brand, a logo of a favourite sports team, a company brand or similar patterns. Employing such structured patterns may enhance the personalization of the aerosol generating device in which the LED module 10 might be used.

It may be further possible to provide the housing with a partially translucent bottom, such the light emitted be the LED or LEDs may be partially transmitted into the housing to control the flow of vapour within the device.

The LED module 10 may have a power supply connector which is configured to be connected to a connection interface of an aerosol generating device 1. The aerosol generating device 1 may comprise an electrical power source, such as a battery, which may be connected to the power supply connector of the LED module 10. Then, the power supply connector may route electrical power input at the connection interface to the at least one LED of the LED module 10 for providing the LED or LEDs with electrical power.

Fig. 2B schematically illustrates a component 2 of an aerosol generating device 1 having an LED module 10 integrated therein. The component may for example be a vaporizer or vaporizing module with an attached LED module 10, a battery or battery module with an integrated LED module 10 (not falling under the scope of the present invention as defined by the appended claims), a flavourant capsule having an LED module 10 integrated (not falling under the scope of the present invention as defined by the appended claims), or a mouthpiece for an aerosol generating device 1 with an LED module 10. Preferably, the component 2 further comprises a particle size measurement device which is capable of measuring or determining the particle size of vapour generated in the aerosol generating device and/or in the surrounding and of adjusting the wavelength of the light emitted by the LED(s) of the LED module 10. The component 2 may in particular comprise a connector for connecting the component 2 to an aerosol generating device, such as the aerosol generating device 1 of Fig. 1.

Fig. 2C schematically illustrates a component 2 of an aerosol generating device 1 having two LED modules 10 integrated therein. The two LED modules 10 may for example comprise LEDs configured to emit light of differing wavelengths. The component 2 of Fig. 2C is only exemplarily shown as comprising two LED modules 10, however, any other number greater than two is also possible for the LED modules 10.

Fig. 3 schematically illustrates a method M for illuminating vapour, especially vapour generated with an aerosol generating device, such as the aerosol generating device 1 of Fig. 1. The method M may comprise as first step M1 emitting light having a wavelength in the range between 465 nm and 485 nm, particularly 470 nm, from at least one light emitting diode, LED, arranged in an aerosol generating device 1. In a second step M2, the emitted light may be directed through vapour generated by the aerosol generating device 1.

Preferably, the method M further comprises a step of measuring the particle size of the vapour particles generated by the aerosol generating device 1 to adjust the wavelength of the light L depending on this particle size and improve light scattering effect.

In the foregoing detailed description, various features are grouped together in one or more examples or examples with the purpose of streamlining the disclosure. It is to be understood that the above description is intended to be illustrative, and not restrictive.

The embodiments were chosen and described in order to best explain the principles of the invention and its practical applications, to thereby enable others skilled in the art to best utilize the invention and various embodiments with various modifications as are suited to the particular use contemplated. In the appended claims and throughout the specification, the terms "including" and "in which" are used as the plain-English equivalents of the respective terms "comprising" and "wherein," respectively. Furthermore, the terms "a" and "an" used herein are intended to be understood as meaning one or more unless explicitly stated otherwise. Moreover, the terms "first", "second", "third", etc. are used merely as labels, and are not intended to impose numerical requirements on or to establish a certain ranking of importance of their objects. Terminology used hereinabove to specify geometric or spatial orientation such as "left", "right", "top", "bottom", "side", "front", "back" and the like is not intended to impose any specific limitation as to the orientation of the objects denoted but rather serve to more easily identify the respective features in the drawings.

### List of Reference Signs

- 1: Aerosol generating device
- 2: Component for an aerosol generating device
- 10: LED module
- 11: Heating element
- 12: Mouthpiece
- 20: User environment
- V0: Vapour stream
- V1: Vapour stream
- L: Light
- M: Method
- M1: Method step
- M2: Method step
- S: Spectrum

## Claims

1. A component for an aerosol generating e-cigarette or a heat-not-burn tobacco aerosol generating device (1), including a light emitting module (10) comprising at least one light emitting device, preferably a light emitting diode, LED, configured to emit light rays (L) having a wavelength in the range between 465 nm and 485 nm;
wherein the component is a mouthpiece or a vapourizing module of the aerosol generating device (1),
wherein the at least one light emitting device is arranged in a substantially cylindrical housing and wherein the at least one light emitting device is configured to emit the light rays (L) substantially perpendicular to the lateral surface of the cylindrical housing,
such that the emitted light rays (L) illuminate vapour generated by the aerosol generating device (1).

2. The component according to claim 1, the light emitting module (10) comprising a power supply connector configured to be connected to a connection interface of an electrical power supply device and configured to route electrical power input at the connection interface to the at least one light emitting device.

3. The component according to claim 1 or claim 2, wherein the at least one light emitting device is configured to emit light rays (L) having a wavelength of 480 nm.

4. The component according to one of the claims 1 to 3, wherein the module comprises means for altering optical diffusion and/or transmission of the light rays (L) emitted by the at least one light emitting device.

5. The component (2) according to one of the claims 1 to 4, further comprising:
a particle measurement device configured to determine the particle size of nearby vapour and to adjust the wavelength of the light rays (L) emitted by the at least one light emitting device depending on the determined particle size.

6. An aerosol generating e-cigarette or a heat-not-burn tobacco aerosol generating device (1), comprising a component (2) according to any of claims 1 to 5.

7. A method (M) for illuminating vapour, comprising:
emitting (M1) light (L) having a wavelength in the range between 465 nm and 485 nm from at least one light emitting device, preferably a light emitting diode, LED, arranged in a substantially cylindrical housing of a mouthpiece or a vapourizing module of an aerosol generating e-cigarette or a heat-not-burn tobacco aerosol generating device (1), wherein the light (L) is emitted substantially perpendicular to the lateral surface of the cylindrical housing; and
directing (M2) the emitted light (L) through vapour generated by the aerosol generating device (1), such that the emitted light (L) illuminates the generated vapour.

8. The method of claim 7, wherein the wavelength of the light (L) is adjusted depending on the particle size of the vapour being measured by a particle measurement device.

9. The method (M) according to any of claims 7 or 8, wherein the particle size of the vapour particles generated by the aerosol generating device (1) is measured and the wavelength of the emitted light is adjusted depending on the measured particle size.

## Patentansprüche

1. Komponente für eine Aerosol erzeugende E-Zigarette oder eine mit nichtverbrennender Tabakerhitzung arbeitende Aerosol erzeugende Einrichtung (1), die ein Licht emittierendes Modul (10) beinhaltet, das mindestens eine Licht emittierende Einrichtung, bevorzugt eine Licht emittierende Diode, LED, umfasst, die dafür ausgelegt ist, Lichtstrahlen (L) mit einer Wellenlänge im Bereich zwischen 465 nm und 485 nm zu emittieren,
wobei es sich bei der Komponente um ein Mundstück oder Verdampfungsmodul der Aerosol erzeugenden Einrichtung (1) handelt,
wobei die mindestens eine Licht emittierende Einrichtung in einem im Wesentlichen zylindrischen Gehäuse angeordnet ist und wobei die mindestens eine Licht emittierende Einrichtung dafür ausgelegt ist, die Lichtstrahlen (L) im Wesentlichen senkrecht zur Mantelfläche des zylindrischen Gehäuses zu emittieren, derart, dass die emittierten Lichtstrahlen (L) von der Aerosol erzeugenden Einrichtung (1) erzeugten Dampf beleuchten.

2. Komponente nach Anspruch 1, wobei das Licht emittierende Modul (10) einen Stromversorgungsanschluss umfasst, der dafür ausgelegt ist, mit einer Verbindungsschnittstelle einer Stromversorgungseinrichtung verbunden zu werden, und dafür ausgelegt ist, an der Verbindungsschnittstelle eingespeisten Strom zu der mindestens einen Licht emittierenden Einrichtung zu leiten.

3. Komponente nach Anspruch 1 oder Anspruch 2, wobei die mindestens eine Licht emittierende Einrichtung dafür ausgelegt ist, Lichtstrahlen (L) mit einer Wellenlänge von 480 nm zu emittieren.

4. Komponente nach einem der Ansprüche 1 bis 3, wobei das Modul Mittel zum Verändern einer optischen Streuung und/oder Transmission der von der mindestens einen Licht emittierenden Einrichtung emittierten Lichtstrahlen (L) umfasst.

5. Komponente (2) nach einem der Ansprüche 1 bis 4, ferner umfassend:
eine Partikelmesseinrichtung, die dafür ausgelegt ist, die Partikelgröße von in der Nähe befindlichem Rauch zu bestimmen und die Wellenlänge der von der mindestens einen Licht emittierenden Einrichtung emittierten Lichtstrahlen (L) abhängig von der bestimmten Partikelgröße anzupassen.

6. Aerosol erzeugende E-Zigarette oder mit nichtverbrennender Tabakerhitzung arbeitende Aerosol erzeugende Einrichtung (1), welche eine Komponente (2) nach einem der Ansprüche 1 bis 5 umfasst.

7. Verfahren (M) zum Beleuchten von Dampf, umfassend:
Emittieren (M1) von Licht (L) mit einer Wellenlänge im Bereich zwischen 465 nm und 485 nm aus mindestens einer Licht emittierenden Einrichtung, bevorzugt einer Licht emittierenden Diode, LED, die in einem im Wesentlichen zylindrischen Gehäuse eines Mundstücks oder eines Verdampfungsmoduls einer Aerosol erzeugenden E-Zigarette oder einer mit nichtverbrennender Tabakerhitzung arbeitenden Aerosol erzeugenden Einrichtung (1) angeordnet ist,
wobei das Licht (L) im Wesentlichen senkrecht zur Mantelfläche des zylindrischen Gehäuses emittiert wird, und
Lenken (M2) des emittierten Lichts (L) durch von der Aerosol erzeugenden Einrichtung (1) erzeugten Dampf, derart, dass das emittierte Licht (L) den erzeugten Dampf beleuchtet.

8. Verfahren nach Anspruch 7, wobei die Wellenlänge des Lichts (L) abhängig von der durch eine Partikelmesseinrichtung gemessenen Partikelgröße des Dampfs angepasst wird.

9. Verfahren (M) nach einem der Ansprüche 7 oder 8, wobei die Partikelgröße der von der Aerosol erzeugenden Einrichtung (1) erzeugten Dampfpartikel gemessen und die Wellenlänge des emittierten Lichts abhängig von der gemessenen Partikelgröße angepasst wird.

## Revendications

1. Composant pour une cigarette électronique produisant des aérosols ou un dispositif produisant des aérosols par chauffage sans combustion du tabac (1), comprenant un module d'émission de lumière (10) comprenant au moins un dispositif d'émission de lumière, de préférence une diode électroluminescente, LED, configurée pour émettre des rayons lumineux (L) ayant une longueur d'onde dans la plage comprise entre 465 nm et 485 nm ;
dans lequel le composant est un embout ou un module de vaporisation du dispositif produisant des aérosols (1),
dans lequel ledit au moins un dispositif d'émission de lumière est agencé dans un récipient substantiellement cylindrique, et dans lequel ledit au moins un dispositif d'émission de lumière est configuré pour émettre les rayons lumineux (L) de manière substantiellement perpendiculaire à la surface latérale du récipient cylindrique, de sorte que les rayons lumineux (L) émis illuminent la vapeur produite par le dispositif produisant des aérosols (1).

2. Composant selon la revendication 1, le module d'émission de lumière (10) comprenant un connecteur d'alimentation électrique configuré pour être connecté à une interface de connexion d'un dispositif d'alimentation électrique et configuré pour acheminer de l'énergie électrique entrée au niveau de l'interface de connexion jusqu'audit au moins un dispositif d'émission de lumière.

3. Composant selon la revendication 1 ou 2, dans lequel ledit au moins un dispositif d'émission de lumière est configuré pour émettre des rayons lumineux (L) présentant une longueur d'onde de 480 nm.

4. Composant selon l'une quelconque des revendications 1 à 3, dans lequel le module comprend des moyens pour modifier la diffusion optique et/ou la transmission des rayons lumineux (L) émis par ledit au moins un dispositif d'émission de lumière.

5. Composant (2) selon l'une quelconque des revendications 1 à 4, comprenant en outre :
un dispositif de mesure de particule configuré pour déterminer la taille de particule de la vapeur proche et pour régler la longueur d'onde des rayons lumineux (L) émis par ledit au moins un dispositif d'émission de lumière en fonction de la taille de particule déterminée.

6. Cigarette électronique produisant des aérosols ou dispositif produisant des aérosols par chauffage sans combustion du tabac (1), comprenant un composant (2) selon l'une quelconque des revendications 1 à 5.

7. Procédé (M) permettant d'illuminer de la vapeur, comprenant les étapes consistant à :
émettre (M1) une lumière (L) ayant une longueur d'onde dans la plage comprise entre 465 nm et 485 nm à partir d'au moins un dispositif d'émission de lumière, de préférence d'une diode électroluminescente, LED, agencée dans un récipient substantiellement cylindrique d'un embout ou d'un module de vaporisation d'une cigarette électronique produisant des aérosols ou d'un dispositif produisant des aérosols par chauffage sans combustion du tabac (1), dans lequel la lumière (L) est émise de manière substantiellement perpendiculaire à la surface latérale du récipient cylindrique ; et
diriger (M2) la lumière émise (L) à travers la vapeur produite par le dispositif produisant des aérosols (1), de sorte que la lumière émise (L) illumine la vapeur produite.

8. Procédé selon la revendication 7, dans lequel la longueur d'onde de la lumière (L) est réglée en fonction de la taille de particule de la vapeur mesurée par un dispositif de mesure de particule.

9. Procédé (M) selon l'une quelconque des revendications 7 ou 8, dans lequel la taille de particule des particules de vapeur produites par le dispositif produisant des aérosols (1) est mesurée et la longueur d'onde de la lumière émise est réglée en fonction de la taille de particule mesurée.
